# EUROPEAN PATENT SPECIFICATION

(11) **EP 1 217 981 B1**
(45) Date of publication and mention of the grant of the patent: **02.11.2006**
(21) Application number: 00968383.0
(22) Date of filing: 21.09.2000
(51) Int. Cl.: A61J 1/00, A61K 41/00

(54) **Container for electrically preparing injectable fluids**
Behälter zur elektrischen Präperation injizierbaren Flüssigkeiten
Récipient de préparation électrique de fluides injectables

(30) Priority: 21.09.1999 US 155465 P
(43) Date of publication of application: 03.07.2002
(60) Divisional application: 06002913.9
(73) Proprietor: Orton, Kevin R., San Clemente, CA 92673 (US)
(72) Inventor: Orton, Kevin R., San Clemente, CA 92673 (US)
(74) Representative: Fiener, Josef
(86) International application number: PCT/US2000/026016
(87) International publication number: WO 2001/021132

(56) References cited:
- DE-A- 1 491 832
- GB-A- 2 074 545
- GB-A- 2 231 961
- GB-A- 2 276 544
- US-A- 5 135 485
- US-A- 5 885 241

## Description

### BACKGROUND OF THE INVENTION

### Field of the Invention

The present invention generally relates to devices used to prepare therapeutic substances. More specifically, the present invention relates to a container according to the pre-characterizing portion of claim 1, used to prepare and dispense electrically-activated therapeutic substances.

### Related Art

One type of biotherapy generally uses an electrolytic fluid that is treated with electrical currents prior to use. In some applications, the fluid is injected into the recipient. The application of the electrical current to the fluid generally is done with a power supply and a signal generator. The electrical current is designed to trigger or enhance the therapeutic effectiveness of the electrolytic fluid or its components.

In general, the electrolytic fluid is placed in a beaker, which holds the fluid and two or more electrodes. A signal generator passes an electrical current through the fluid between the electrodes to condition the fluid. After application of the current, the fluid is filtered and sterilized as needed before being placed in a syringe for injection into the patient. The patient is usually a human or mammal.

With reference now to Figure 1, conventional preparation equipment, as disclosed in US-A-5,885,241, which includes a beaker 10, is illustrated. The beaker 10 includes a reservoir area 12 for fluids, a set of electrodes 14 that extend into the reservoir area 12 and an opening area 16. Fluids 18 may be placed in the container 10 and an electric current can be applied to the fluids 18 through a set of wires 20 and the corresponding electrodes 14. The current can be supplied by a signal generator 22. When the application of electric current is completed, a utensil 24, such as a syringe, a tube, a squeeze bulb, a pipette, or any other suitable utensil, can be used to remove the electrically prepared fluid from the container 10. The fluid then is prepared for injection.

This process for preparing the active fluid has several disadvantages. One disadvantage is that the fluid must be handled quite a bit, such as when it is transferred between generally open containers to prepare it for use and to prepare it for administration. Additionally, the electrodes and the activating container must be cleaned and sterilized before each use, which activities are time consuming and require specialized equipment.

Therefore, a simpler, easier method of electrically preparing substances for injection is desired.

### SUMMARY OF THE INVENTION

Accordingly, a non-round container has been developed for preparing and administering electrically prepared substances used by injection without requiring a transfer of the substances from one container to another container, as defined in claim 1.

A preferred embodiment of the present invention involves a flexible container adapted to hold a fluid. The container comprises a flexible wall that at least partially defines a reservoir. At least two electrically conductive electrodes are disposed at least partially in the reservoir. A gap is defined between the electrodes and the electrodes each have a portion exposed outside of the container when the container is closed.

### BRIEF DESCRIPTION OF THE DRAWINGS

These and other features, aspects and advantages of the present invention will now be described with reference to the drawings of a couple of preferred embodiments, which embodiments are intended to illustrate and not to limit the invention, and in which figures:

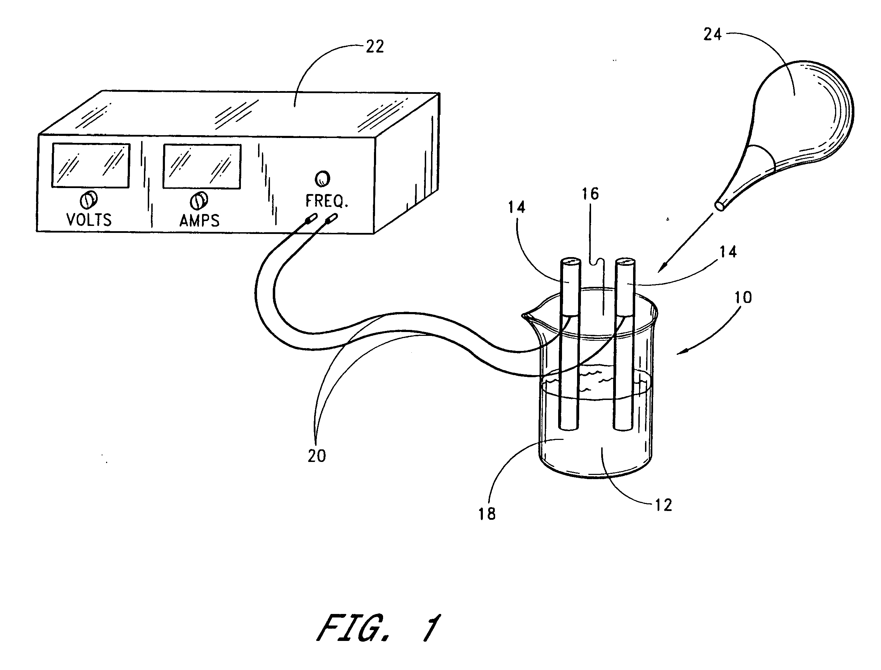

Figure 1 is a schematic illustration of traditional containers used in preparing an electrically prepared fluid;
Figure 2 is a side elevation view of a container constructed according to certain features, aspects and advantages of the present invention;
Figure 3 is an enlarged view of a seal of the container of Figure 2; and
Figure 4 is a schematic view of another container, which is used for intravenous feed and which also is constructed in accordance with certain features, aspects and advantages of the present invention.

### DETAILED DESCRIPTION OF THE PREFERRED EMBODIMENTS OF THE INVENTION

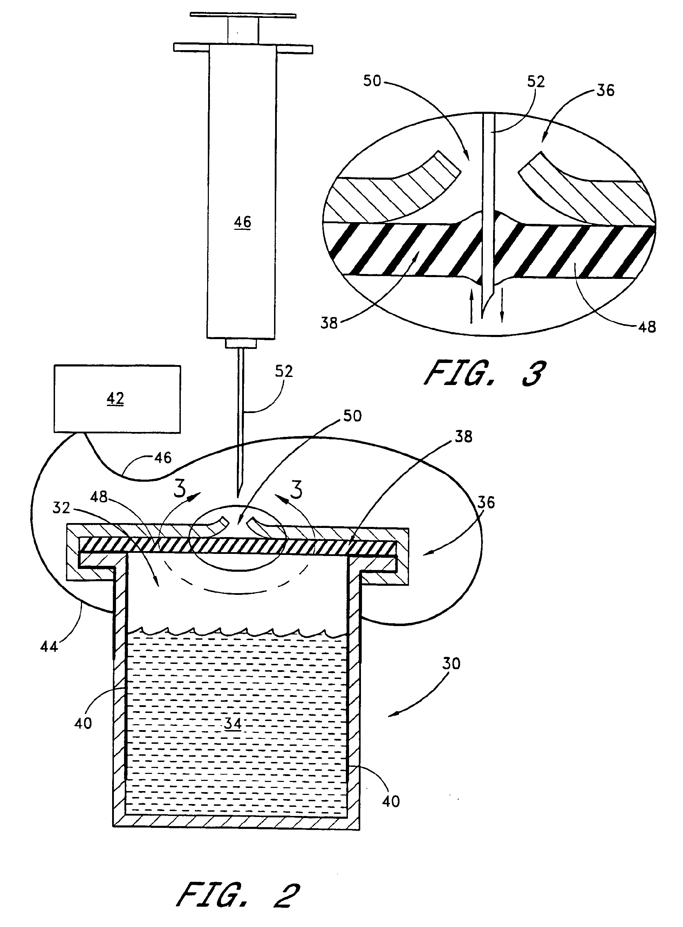

With reference now to Figure 2, a container having certain features, aspects and advantages in accordance with the present invention is illustrated. Preferably, the container 30 is a relatively small size. For example, in one arrangement, the container 30 is designed to hold between about 1 milliliter and 1 liter of fluid. In other arrangements, the container 30 can be handled with exiting automated filling and assembly equipment. In some particularly advantageous arrangements, the container 30 is sized to accommodate a single dosage of electrically-prepared fluid.

With continued reference to Figure 2, a reservoir area 32 is defined within the container 30. In the illustrated arrangement, the container 30 accommodates any suitable injectable grade fluids 34 and the reservoir 32 is closed with a lid 36. A seal 38 preferably is interposed between a portion of the lid 36 and a portion of the container 30. In any event, the lid 36 desirably is tightly sealed to the container 30 and is configured to resist opening. In one arrangement, the lid 36 is designed to provide evidence of tampering after the lid 36 is secured to the container 30. Of course, the sealing construction, the tamper resistant construction and the tamper evidencing construction can be of any suitable configuration and many such constructions are well known.

Electrically conductive electrodes 40 are at least partially disposed within the reservoir area 32. The electrodes 40 are directly secured to an inner surface of the container 30. The electrodes 40 are positioned such that they are in direct contact with at least a portion of the fluid 34 disposed within the container 30. While the illustrated electrodes 40 are placed on the sides of the container 30, the electrodes 40 can be placed on the ends of the container, while maintaining contact with the fluid 34 disposed within the container 30.

With continued reference to Figure 2, the electrodes 40 are positioned on opposing inner surfaces of the container 30. By positioning the electrodes 40 on opposing inner surfaces of the container 30, the separation of the electrodes 40 can be maximized.

The electrodes 40 extend outside of the reservoir area 32. The electrodes 40 preferably are formed of gold, titanium or other suitable metals or conductive elements. The electrodes 40 can be formed with electrodeposits of vapors, by electroplating, with metal foil tape or by any other suitable techniques. Preferably, the electrodes 40 are thin enough to not interfere with the seal 38 that is disposed between the lid 36 and the container 30. Of course, the electrodes 40 can be enlarged if an electrical connection can be established in other methods. For instance, the electrodes 40 can extend through at least a portion of the lid 36. In one presently preferred arrangement, the electrodes 40 generally are about 44 mm wide and about .30 mm thick. The exact sizing and configuration of the electrodes 40 can vary; however, the electrodes 40 preferably have a resistance below approximately 5000 ohms per square centimeter, and insulating portions disposed between the electrodes 40 should provide approximately 100 ohms or more resistance between the electrodes 40 to avoid shorting away excessive current.

In the illustrated arrangement, the seal 38 is configured in such a manner to provide a substantially contamination resistant seal between the contents of the container 30 and the exterior of the container 30. In addition, the electrodes 40 extend from the interior of the container 30, past the seal 38 to the outside of the container 30, where clip leads or other suitable electrical connections may be made.

The container 30 can be filled under controlled conditions using suitable processes. In one arrangement, the container 30 is filled with a sterile, injectable fluid 34 prior to the lid 36 being secured in position on the container 30. The processes advantageously produce a filled container 30 that has sterilized and relatively particulate free contents. In some applications, it is particularly important that the container 30 be free of even sterile particulate matter, in order to avoid an adverse reaction in the patient.

Prior to administration, a current can be applied to the container 30. In the illustrated arrangement, a signal generator 42 is used to supply the current. Various types of electrical signal generators 42 may be used to provide the electric signals. Genetronics, Inc., of San Diego, CA makes several bioelectric signal-generating apparatuses that can be used, for instance. The current preferably is applied for a preset period of time before any activated fluid 34 is withdrawn from the container 30.

The current travels from one wire 44 of the signal generator 42 to a clip (not shown). At the clip, the current is transferred to an exterior portion of one electrode 40. Of course, other electrical connections can result in the current passing through an intermediate member before passing to the electrode 40. In addition, various electrical connectors, plugs, or sockets, clip leads or other fittings may be used to provide electrical connection between the external portion of the electrode 40 and the electrical signal generator 42.

Once the current has flowed into the electrode 40, the current passes through the contents 34 of the container 30 to the second electrode 40. The current then is returned to the signal generator 42 or to a grounded connection by passing through a second wire 46. Additional electrodes (i.e., more than 2) can be used in some applications.

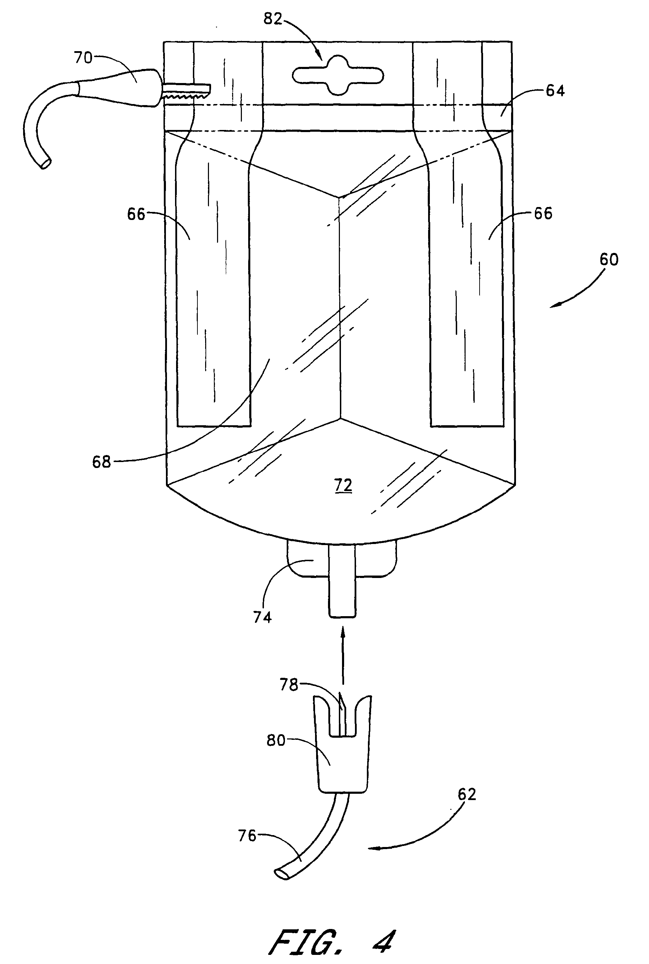

In the illustrated arrangement, the seal 38 comprises a barrier member 48 that allows the contents of the container 34 to be directly transferred to an applicator 46, such as a syringe, a catheter, a hypodermic needle, or any other suitable device, in a sterile manner. The member 48 can be any suitable septa. In some applications, the barrier member 48 comprises a soft type of elastomeric or rubber type material that is incorporated into a part of the container 30 or lid 36. For instance, the member 48, in combination with an access opening 50 formed in the lid 36, can provide a liquid and contamination tight seal while allowing a hypodermic needle 52 to temporarily pierce the seal 38 (see Figure 4). The needle 52 then can extract the electrically prepared fluid 34 from the container. Upon withdrawal of the needle 52, the barrier member 48 preferably reseals the opening through which the needle 52 was inserted. Thus, the barrier member 48 maintains a relatively contamination resistant seal for the contents of the container 30 while the syringe or other device is withdrawing activated fluids 34. Any suitable barrier member construction can be used.

With reference now to Figure 4, another arrangement of the container 60 is illustrated therein. As illustrated, the container 60 can be formed of a flexible vinyl-type material. In the illustrated configuration, the container 60 is a bag; however, other flexible designs (i.e., pouches, envelopes, etc.) also can be used. The illustrated bag 60 is configured and used to supply an intravenous feed 62. In addition, in the illustrated arrangement, the bag 60 is heat-sealed 44 at a seal line 64 after the fluids have been added. Preferably, one lip of the bag 60 is shorter than the other lip such that the longer lip extends a greater distance above the seal line 64 than the shorter lip for reasons that will become apparent. As will be recognized, no separate lid is needed in this arrangement.

With continued reference to Figure 4, a set of electrodes 66 preferably are at least partially disposed within the bag 60. In the illustrated arrangement, the electrodes 66 advantageously are formed on the interior walls of the bag 60. It should be recognized that the electrodes can be integrally formed with the walls 68 of the bag 60 in some applications such that the electrodes 66 might be placed within electrical communication with the contents of the bag 60 while also including a portion of the electrode 66 which is exposed to the exterior of the bag. In the illustrated arrangement, a portion of each electrode 66 is in electrical contact with the contents of the bag 60 and another portion of each electrode 66 passes through the seal line 64 of the bag 60 to the exterior of the bag 60. An electrical connection 70, such as a clip that is used in the illustrated arrangement, can be attached to the portion of the electrode 66 that is outside of the bag 60.

The electrodes 66 in the illustrated arrangement are positioned along opposing portions of the bag 60. The electrodes 66 can be of any suitable size and configuration. The electrodes 66 are generally rectangular in shape. In addition, the electrodes 66 can be formed by various techniques. One such technique is to use a metalized foil strip laminated on to a vinyl substrate. The laminated metal then is incorporated into the construction of the bag 60.

A portion of the container 60, generally at a lower end 72 in the illustrated arrangement, preferably comprises one or more openings or fittings 74. The fittings 74 can be adapted to connect with a tube 76 or a hypodermic needle 78 such that the fluid contained within the bag 60 can be drawn outward in a controlled and sterile manner. In the illustrated configuration, the act of coupling the external tube 76 to the fitting 74 on the container 60 will break a seal portion on the container 60. For instance, the hypodermic needle 78 can be integrated to a fitting 80 that connects with the fitting 74 of the bag 60. The needle 78 can pierce the seal and extend into the fluid reservoir area. The fitting 80 may take various forms, with a standard Luer type medical fitting providing acceptable results.

Preferably, the bag 60 is adapted to provide an intravenous feed supply. The vinyl portion can include any of a number of suitable mechanisms 82 for hanging the bag from a hook or other apparatus. In addition, the opening or fitting 74 can be located at the lower end 72 of the bag 60 to-provide gravity feed of the fluid from within the reservoir area. In some applications, a motorized pump or power feed mechanism can be used. Desirably, the bag 60 is configured so that at least a portion of the bag 60 is flexible and will collapse as the contents of the bag 60 are withdrawn, thereby preventing the entry of air into the bag 60 and maintaining a substantially controlled egress of fluid from the bag 60.

Several different types of operations can be performed on the contents of the container 30 or bag 60 fitted with the electrodes 40, 66. In some arrangements, the ionic properties of the fluid can be altered. In other arrangements, the ph level of the contents can be changed. Of course, other effects can be stimulated in suitable manners.

Preferably, in both arrangements, the container is filled and sterilized in a single location, using any suitable processes and any suitable automated equipment. The filled and sterilized container then can be transferred to the administration site. As will be appreciated, one aspect of the present invention provides that the substance within the container can be electrically prepared without need of removing it from the container prior to withdrawing at least a portion for injection. This aspect results in less transfer of the substance and improved sterility of the substance up until the moment of withdrawal for injection. In other words, there is minimal handling and minimal chance of contamination to the substance before, during, and after the time it is administered. Thus, this invention provides a construction whereby a sterile substance can be electrically prepared with minimal chance of contamination prior to injection. Thus, this device provides an easier to use, more sterile and more reliable method for administering electrically sensitive substances. It should be noted that, while the substance within the container may be injected, it need not be. The substance also may be applied topically or externally.

Although the present invention has been described in terms of certain preferred embodiments, other embodiments apparent to those of ordinary skill in the art also are within the scope of this invention. Thus, various changes and modifications may be made without departing from the scope of the invention. For instance, various components may be repositioned as desired. Moreover, not all of the features, aspects and advantages are necessarily required to practice the present invention. Accordingly, the scope of the present invention is intended to be defined only by the claims that follow.

## Claims

1. A container (30, 60) for preparing an electrically activated fluid (34) by application of an electrical signal (42) so as to act on the fluid (34) for use as a medicament,
said container (30, 60) including an electrolytic fluid (34) and comprising walls (68), a reservoir (32) defined at least in part by the walls (68), and at least two electrically conductive members (40, 66) extending into the reservoir (32),
the electrically conductive members (40, 66) not contacting one another within the reservoir (32), the electrolytic fluid (34) constituting an electrically conductive path where current passes through the fluid (34) in the container (30) between the electrically conductive members (40, 66),
the electrically conductive members (40, 66) being generally rectangular in shape and being arranged such that they extend along the inner surface of the container (30, 60), and
the conductive members (40, 66) being in electrical communication with contact elements (70) outside the reservoir (32),
**characterized in that**
the container (30, 60) is a non-round container,
the electrolytic fluid (34) is an injectable grade fluid,
the electrically conductive members (40, 66) are positioned on or directly secured to opposing inner surfaces of the container (30, 60), and
a sealing portion (38, 64) cooperating with the walls (68) encloses the reservoir (32).

2. The container of Claim 1, wherein the sealing portion (38) is a lid fitted with a soft pierceable member adapted to facilitate transfer of fluid (34) out of the reservoir (32) into a hypodermic needle (52, 78), in a contamination resistant manner.

3. The container of Claim 2, wherein the container (30, 60) comprises a fitting (74).

4. The container of any of Claims 1 - 3, wherein the container (60) is a bag.

## Patentansprüche

1. Behälter (30, 60) zur Präparation eines elektrisch aktivierten Fluids (34) durch Anlegen eines elektrischen Signals (42) zur Einwirkung auf das Fluid (34) zur Verwendung als Medikament, wobei
der Behälter (30, 60) ein elektrolytisches Fluid (34) einschließt und Wände (68), ein zumindest zum Teil von den Wänden (68) begrenztes Reservoir (32) und zumindest zwei sich in das Reservoir (32) hinein erstreckende elektrisch leitende Teile (40, 66) umfasst,
die elektrisch leitenden Teile (40, 66) einander im Innern des Reservoirs (32) nicht berühren,
das elektrolytische Fluid (34) einen elektrisch leitenden Weg bildet, wo Strom zwischen den elektrisch leitenden Teilen (40, 66) durch das Fluid (34) in dem Behälter (30) fließt, die elektrisch leitenden Teile (40, 66) von allgemein rechteckiger Form sind und derart angeordnet sind, dass sie entlang der Innenfläche des Behälters (30, 60) verlaufen, und
die leitenden Teile (40, 66) mit Kontaktelementen (70) außerhalb des Reservoirs (32) in elektrischer Verbindung stehen,
**dadurch gekennzeichnet, dass**
der Behälter (30, 60) ein nicht runder Behälter ist,
das elektrolytische Fluid (34) ein Fluid einer injizierbaren Art ist,
die elektrisch leitenden Teile (40, 66) an gegenüberliegenden Innenflächen des Behälters (30, 60) positioniert oder direkt daran befestigt sind, und
ein mit den Wänden (68) zusammenwirkender Dichtungsabschnitt (38, 64) das Reservoir (32) umschließt.

2. Behälter nach Anspruch 1, worin der Dichtungsabschnitt (38) ein Deckel ist, der mit einem weichen durchbohrbaren Teil ausgestattet ist, das geeignet ist, eine Übertragung von Fluid (34) kontaminationsresistent aus dem Reservoir (32) in eine Nadel (52, 78) zur subkutanen Injektion zu erleichtern.

3. Behälter nach Anspruch 2, worin der Behälter (30, 60) ein Anschlussteil (74) umfasst.

4. Behälter nach einem der Ansprüche 1 bis 3, worin der Behälter (60) ein Beutel ist.

## Revendications

1. Récipient (30, 60) de préparation d'un fluide activé électriquement (34) par l'application d'un signal électrique (42) de façon à agir sur le fluide (34) destiné à être utilisé comme médicament,
ledit récipient (30, 60) comprenant un fluide électrolytique (34) et étant composé de parois (68), d'un réservoir (32) défini au moins en partie par les parois (68), et d'au moins deux éléments électriquement conducteurs (40, 66) s'étendant dans le réservoir (32),
les éléments électriquement conducteurs (40, 66) n'étant pas en contact l'un avec l'autre dans le réservoir (32),
le fluide électrolytique (34) constituant un chemin électriquement conducteur où le courant passe à travers le fluide (34) dans le récipient (30) entre les éléments électriquement conducteurs (40, 66),
les éléments électriquement conducteurs (40, 66) étant de forme globalement rectangulaire et étant agencés de façon à s'étendre le long de la surface intérieure du récipient (30, 60), et
les éléments conducteurs (40, 66) étant en communication électrique avec des éléments de contact (70) à l'extérieur du réservoir (32),
**caractérisé en ce que**
le récipient (30, 60) est un récipient non-rond,
le fluide électrolytique (34) est un fluide de qualité injectable,
les éléments électriquement conducteurs (40, 66) sont positionnés ou directement fixés sur des surfaces intérieures opposées du récipient (30, 60), et
une partie d'étanchéité (38, 64) coopérant avec les parois (68) ferme le réservoir (32).

2. Récipient selon la revendication 1, dans lequel la partie d'étanchéité (38) est un couvercle pourvu d'un élément perçable mou conçu pour faciliter le transfert de fluide (34) du réservoir à une aiguille hypodermique (52, 78), d'une manière résistante à la contamination.

3. Récipient selon la revendication 2, dans lequel le récipient (30, 60) comprend un raccord (74).

4. Récipient selon l'une quelconque des revendications 1 à 3, dans lequel le récipient (60) est une poche.
